(19) Europäisches Patentamt
European Patent Office
Office européen des brevets

(11) EP 0 737 200 B1

(12) **FASCICULE DE BREVET EUROPEEN**

(45) Date de publication et mention de la délivrance du brevet: **12.06.2002 Bulletin 2002/24**

(21) Numéro de dépôt: **95933457.4**

(22) Date de dépôt: **29.09.1995**

(51) Int Cl.[7]: **C07F 7/18**, A61K 31/695, A61K 7/48

(86) Numéro de dépôt international: **PCT/FR95/01266**

(87) Numéro de publication internationale: **WO 96/10575 (11.04.1996 Gazette 1996/16)**

(54) **COMPOSES A BASE DE SILICIUM BIOLOGIQUEMENT ACTIFS ET APPLICATIONS**

ZUSAMMENSETZUNGEN AUF DER BASIS VON BIOLOGISCH AKTIVEN SILIZIUM UND IHRE VERWENDUNG

BIOLOGICALLY ACTIVE SILICON COMPOUNDS AND THEIR THERAPEUTIC AND COSMETIC APPLICATIONS

(84) Etats contractants désignés:
**BE CH DE ES GB GR IE IT LI NL PT**

(30) Priorité: **30.09.1994 FR 9412088**

(43) Date de publication de la demande:
**16.10.1996 Bulletin 1996/42**

(73) Titulaire: **EXSYMOL**
**98000 Monaco (MC)**

(72) Inventeurs:
- **SEGUIN, Marie-Christine**
  **MC-98000 Monaco (MC)**
- **GUEYNE, Jean**
  **MC-98000 Monaco (MC)**
- **NICOLAY, Jean-François**
  **F-06230 Villefranche-sur-Mer (FR)**
- **FRANCO, André**
  **F-06500 Menton (FR)**

(74) Mandataire: **Bonneau, Gérard**
**Cabinet Bonneau,**
**Conseil en Propriété Industrielle,**
**Les Taissounières HB3**
**1681, Route des Dolines**
**06560 Sophia Antipolis (FR)**

(56) Documents cités:

| | |
|---|---|
| **EP-A- 0 136 501** | **EP-A- 0 148 398** |
| **EP-A- 0 227 025** | **EP-A- 0 273 266** |
| **EP-A- 0 295 983** | **EP-A- 0 368 481** |
| **EP-A- 0 395 433** | **DE-A- 2 929 225** |
| **DE-A- 3 240 971** | **FR-A- 2 314 724** |
| **FR-A- 2 335 232** | **US-A- 3 546 267** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 737 200 B1

## Description

Domaine technique :

La présente invention concerne des composés à base de silicium biologiquement actifs, notamment au contact des tissus vivants, ainsi que les applications thérapeutiques et cosmétiques de ces composés.

Etat de la technique :

Le silicium, élément très répandu dans la nature est généralement connu sous ses formes inorganiques naturelles telles que la silice et les silicates, ou encore sous la forme de polymères synthétiques, les silicones. Ces composés siliciés sont très peu ou pas solubles en milieu aqueux ce qui explique leur faible incidence au niveau des organismes vivants. Les silicones, en particulier, se caractérisent par une grande inertie vis à vis des milieux biologiques et par conséquent présentent une biocompatibilité élevée.

Pourtant, le silicium, même à l'état de traces, joue un rôle biologique important et doit être considéré comme un élément essentiel de la vie. Il est notamment nécessaire à une croissance normale pour de nombreuses espèces. Il a aussi été démontré que le silicium intervenait dans la structuration des tissus conjonctifs en interagissant avec les glycosaminoglycanes et des protéines. C'est un des éléments constitutifs des complexes protéines-glycosaminoglycanes rencontrés dans la substance fondamentale de ces tissus. Le silicium interagit également avec les glycosaminoglycanes dans le développement des tissus cartilagineux. On sait aussi que le silicium joue un rôle important dans l'ossification où il favorise le processus de minéralisation.

En outre, le silicium peut être considéré comme un constituant du collagène et on pense qu'il joue un rôle fondamental dans le processus de réticulation des fibres de collagène.

Le silicium est également impliqué dans le métabolisme cellulaire et il serait notamment propice à l'activité métabolique des ostéoblastes.

Au delà du pouvoir réticulant du silicium et de son implication dans l'activité métabolique de certaines cellules, il apparaît qu'une teneur élevée en silicium dans les tissus, parallèlement avec la teneur en glycosaminoglycanes, est caractéristique des tissus sains et métaboliquement actifs.

Les recherches actuelles tendent à renforcer l'idée que le silicium intervient dans de nombreux mécanismes biologiques. Des travaux récents ont même montré que le silicium joue un rôle majeur dans l'élimination de l'aluminium par les systèmes biologiques.

Les travaux de la demanderesse ont montré que les composés siliciés pouvaient constituer une forme de silicium assimilable par l'organisme (par opposition au silicium minéral ou aux silicones) à condition de posséder la propriété d'exister en solution aqueuse sous forme d'oligomères solubles de faible poids moléculaire. En outre, une autre caractéristique nécessaire à l'activité de ces oligomères en solution aqueuse est de présenter de nombreuses fonctions Si-OH. Il ressort ainsi que les propriétés biologiques de ces composés siliciés assimilables par l'organisme, ne sont observées que s'ils forment en solution des oligomères solubles, résultant d'un enchaînement de liaisons siloxanes Si-O-Si, riches en fonctions Si-OH.

En dehors du fait que la présence des fonctions Si-OH, très polaires, confèrent aux oligomères leur solubilité dans l'eau, on pense à l'heure actuelle qu'une partie des propriétés observées s'expliquent par le fait que l'espèce chimique impliquée dans la plupart des mécanismes biologiques mentionnés ci-dessus serait une forme soluble du silicium, l'acide silicique, de formule $Si(OH)_4$. Mais, étant donnée sa très forte propension à se polycondenser pour former de la silice, ce composé n'existe qu'à de très faibles concentrations dans l'eau.

On a donc recherché des produits analogues à l'acide silicique, plus stables, en modifiant chimiquement les fonctions Si-OH. Mais il est vite apparu que ces fonctions étaient essentielles à l'activité biologique. Par ailleurs, on savait qu'une série de composés naturels, les tanins et les catécholamines entre autres, étaient capables de complexer l'acide silicique et ainsi d'augmenter sa stabilité en solution. Ces complexes constitueraient la forme de transport de l'acide silicique dans l'organisme et c'est sous cette forme que la cellule importerait le silicium. Toutefois, leur stabilité est encore trop faible pour la réalisation d'un produit pharmacologiquement actif.

On connaît l'utilisation de composés organosilicés comme agents de durcissement se fixant par polymérisation aux extrémités silanol de polymères organosiliciés tels que les éthers d'alcoxysilyls décrits dans le brevet DE 2929225 ou les organosiliciés comportant un groupement vinylique décrits dans la demande EP-A-0.395.433.

On peut encore citer d'autres applications du même type telles que l'utilisation de dialcoxysilylénoléthers comme agents de terminaison dans la synthèse de polysiloxanes (demande DE 3240971) ou encore celle de composés fluorophores comprenant un groupement organosilicié jouant le rôle d'agent de réticulation pour la fixation sur un support de silicone (demande EP-A-0368481).

Le brevet EP-0.227.025 décrit des composés organosiliciés à base de fluor utiles comme agents de revêtement de bandes magnétiques.

Les demandes américaines US-A-4.584.394 et US-A-4.678.283 décrivent respectivement des trialkanoyloxysilanes comme composant de cristaux liquides et des alkénylacyloxysilanes utilisés dans la fabrication d'adhésifs par copolymérisation.

Ces demandes n'entrent pas dans le champ de la présente invention car il n'est pas question d'activité biologique du composé silicié.

Un autre type d'application tel que décrit dans la demande EP-A-0.273.266 consiste à proposer des dérivés siliciés du menthol contenant au moins un radical menthoxy qui s'hydrolysent pour libérer du menthol, principe actif induisant une réponse analgésique. Là encore, il n'est pas question de composés organosiliciés biologiquement actifs stabilisés.

Enfin, la demande EP-A-0.295.983 décrit des composés organosiliciés susceptibles de s'hydrolyser ou de s'alcooliser en solution mais ne décrit ni ne suggère que ces composés s'hydrolysent in vivo en libérant un composé biologiquement actif et un stabilisant.

Exposé de l'invention :

C'est pourquoi le but principal de l'invention est de fournir des composés à base de silicium possédant des liaisons biologiquement hydrolysables notamment au contact des tissus vivants et permettant d'obtenir des oligomères possédant les fonctions Si-OH biologiquement actives et au moins un stabilisant qui stabilise les oligomères ainsi formés.

L'invention a donc pour objet un composé à base silicium libérant par hydrolyse in vivo un composé silylé biologiquement actif et au moins un stabilisant, ledit composé ayant la formule générale suivante:

a) 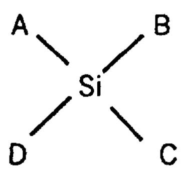

b) 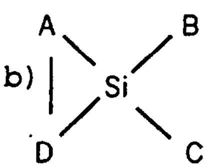

dans laquelle :

- A et D sont radicaux tels que leur produits d'hydrolyse A-H et/ou D-H est un stabilisant choisi dans le groupe consistant en :

  acides carboxyliques simples possédant une chaîne carbonée d'au moins cinq atomes de carbone, phénols, hydroxyacides carboxyliques tels que les alpha et les bêta hydroxyacides, les glucuronides, composés comportant plusieurs fonctions alcools ou phénols et surtout des fonctions alcools ou phénols vicinales tels que les glycols, les catéchols, et les catécholamines, les polyéthylène-glycols, les polyols tels que le glycérol, les monosaccharides tels que le L-thréose, L-ribose ou sorbitol, les acides phénoliques tels que l'acide caféique et leurs dérivés estérifiés, les diacides tels que l'acide malonique, et certains composés possédant une géométrie particulière tels que le tropolone,

- B est un radical tel que son produit d'hydrolyse est choisi parmi les composés que représentent A-H et D-H ou un radical alkyl ou alcoxy,

- C est un radical hydrocarboné tel qu'un groupement alkyle alkényle, alkyne, arylalkyle ou aryle, à l'exception d'un phényle.

Description de l'invention :

Dans le composé de l'invention tel qu'il est défini ci-dessus, les radicaux qui sont biologiquement hydrolysables sont des radicaux liés à Si par un atome d'oxygène et qui libèrent par hydrolyse au moins un stabilisant A-H, B-H et/ou D-H.

Les radicaux liés à Si et qui ne sont pas hydrolysables sont ceux qui présentent une liaison Si-C et peuvent être des radicaux hydrocarbonés tels que des radicaux alkyle, alkényle, alkyne, arylalkyle et aryle à l'exclusion toutefois des radicaux aryle de type phényle. Cette exclusion est justifiée par le fait que les composés de type phényl-Si-OH qui seraient obtenus après hydrolyse de liaisons hydrolysables, présentent des effets néfastes sur les organismes

vivants et sont notamment toxiques vis-à-vis du foie, du pancréas, de la moelle osseuse et du muscle cardiaque (voir l'article "Bioactive silanes et siloxanes" par R.R. Le Vier dans L'actualité Chimique, mars 1986, pages 89-91). Les radicaux non hydrolysables peuvent être aussi des radicaux fluorocarbonés de type fluoroalkyle ou fluoroalkényle.

On doit noter que sont exclus tous les autres types de liaisons biologiquement non hydrolysables et notamment les liaisons Si-O-Si connues pour leur inertie chimique et leur absence de réactivité vis-à-vis des milieux biologiques.

Des exemples de composés selon l'invention sont donnés ci-après :

Dérivés de l'alcool salicylique (ou 2-hydroxybenzylalcool):

2-oxo-benzyloxy-éthoxyméthylsilane

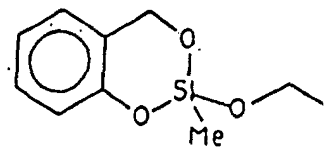

2-oxo-benzyloxy-diméthylsilane

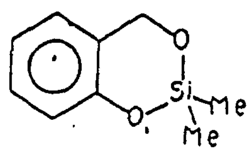

Dérivés d'alpha-hydroxyacides :

Ethoxyméthylsilyl-2-oxo-octanoate

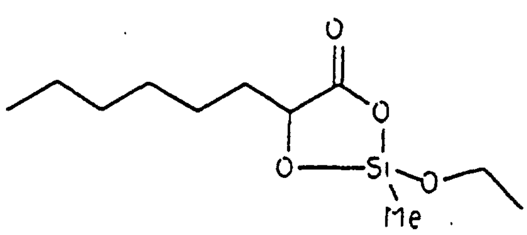

Diméthylsilyl-2-oxo-octanoate

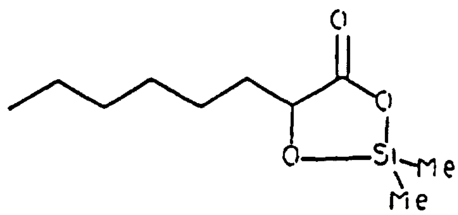

2,2-éthoxyméthyl-4-oxo-5-méthyl-1,3-dioxa-2-cyclosilane ou éthoxyméthylsilyl-lactate :

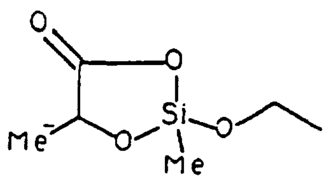

Dérivés d'acides à longue chaîne:

Diméthyldidécanoyloxysilane :

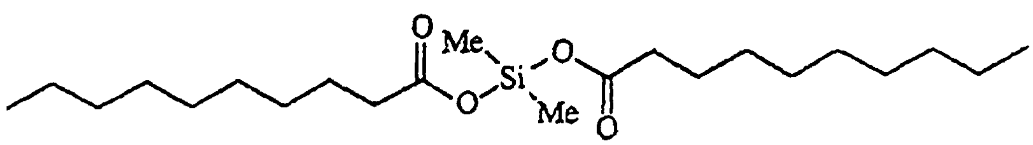

Méthylsilanetridécanoylester

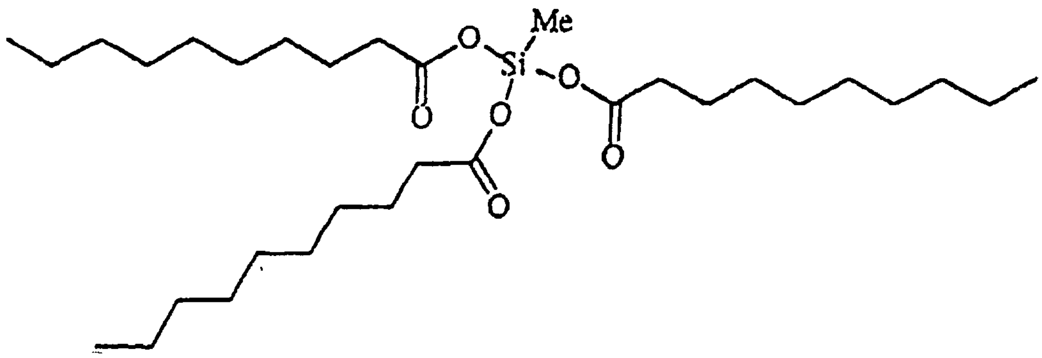

Caproyldiéthoxysilane :

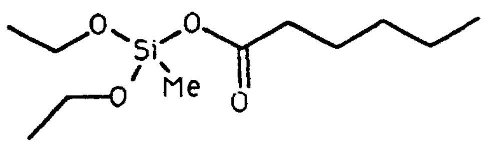

Dérivés de l'acide salicylique :

2,2-méthyl,tertbutoxy-4-oxo-1,3-dioxa-2-silane :

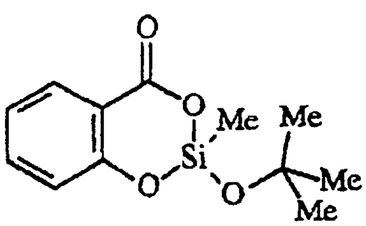

2,2-diméthyl-4-oxobenzo-1,3-dioxa-2-silane :

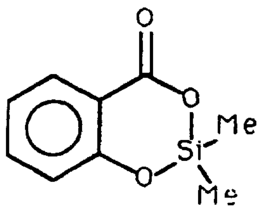

2,2-éthoxy, n-octyl-4-oxobenzo-1,3-dioxa-2-silane :

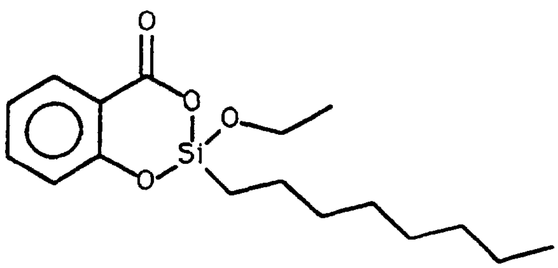

2,2-diméthyl-4-oxo-(3-octanoyl benzo)-1,3-dioxa-2-silane :

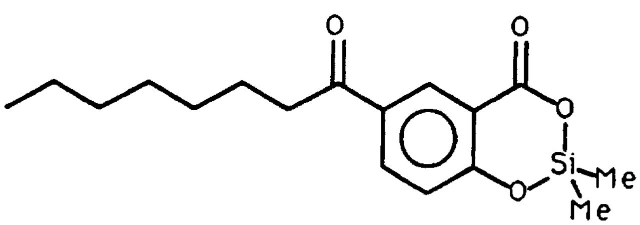

Divers :

**Diméthylsilyl-2,3-5,6-ascorbate :**

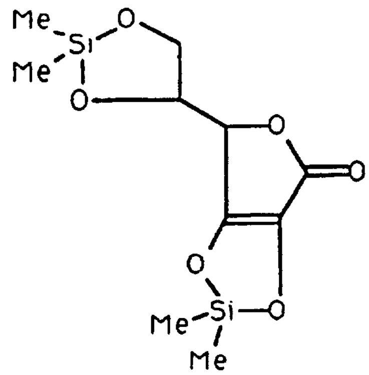

Par la suite, on appellera silyl le composé biologiquement actif comportant deux ou trois liaisons Si-OH, et on admettra que les composés qui font l'objet de l'invention sont des "précurseurs" de silyl appelés plus simplement précurseurs dans le reste de la description.

Les méthodes de préparation des composés selon l'invention sont multiples. Elles utilisent comme produits de départ des silanes fonctionnalisés disponibles dans le commerce tels que des chlorosilanes comme le tétrachlorosilane $SiCl_4$, des alkoxysilanes comme le tétraalkoxysilane $SiOR_4$, des silanes hétéro-fonctionnels comme le trichlorosilane $Cl_3SiH$ ou des alkylsilanes fonctionnalisés afin de réduire le nombre d'étapes de synthèses comme le diméthyldichlorosilane, le méthyltriétoxysilane, etc...

Les précurseurs selon l'invention sont donc des composés biologiquement inactifs, capables, après administration topique, par voie orale ou systémique, de se transformer spontanément ou en présence d'un catalyseur enzymatique spécifique tel que la silicase pour former un composé biologiquement actif.

Si on considère une seule liaison hydrolysable du précurseur (sachant que deux ou trois des liaisons sont hydrolysées de la même manière), la transformation suivante a lieu au contact d'un tissu vivant tel que la peau ou les muqueuses, ou d'un fluide biologique.

$$Si \qquad P + H_2O \rightarrow Si\ OH + P - H$$

Comme il a été mentionné précédemment, les composés -Si-OH se trouvant en milieu aqueux ont tendance à former des oligomères solubles par condensation de deux, trois ou quelques molécules de composés -Si-OH. Il est clair que si ce composé ne comporte qu'une seule liaison OH, une telle condensation aboutissant à des dimères ferait disparaître les liaisons Si-OH. Il faut donc que les précurseurs selon l'invention aient au moins deux liaisons hydrolysables.

Le produit P-H obtenu dans la réaction ci-dessus peut être un composé inactif non toxique ou un composé pouvant favoriser, renforcer ou compléter l'action du silyl.

Une caractéristique commune aux précurseurs, essentielle dans la réalisation de l'invention, est que que le produit P-H obtenu après hydrolyse joue le rôle d'agent stabilisant qui préserve l'activité des silyls en s'opposant à leur polycondensation qui conduirait à la formation de composés insolubles dans l'eau et donc inactifs. Après hydrolyse, l'agent stabilisant est libéré dans le milieu et stabilise le silyl en créant des liaisons faibles avec lui (ponts hydrogènes). Le pouvoir stabilisant de certains de ces agents peut aussi s'expliquer par leur capacité à reformer une liaison covalente transitoire. On obtient alors une structure dynamique où certaines liaisons possèdent un "caractère mixte" (liaison hydrogène, liaison covalente).

Les stabilisants répondant au critère énoncé ci-dessus sont après hydrolyse, des acides carboxyliques simples (dont les esters sont les précurseurs) possédant de préférence une chaîne carbonée supérieure à 5 atomes de carbone, ceci grâce à leur capacité à former des structures micellaires propres à stabiliser les silyls, des phénols ( dont les radicaux phénoxy sont les précurseurs) particulièrement à même de former des liaisons covalentes transitoires, et surtout des composés polyfonctionnels tels que des composés hydroxyacides carboxyliques et particulièrement les

alpha et les bêta hydroxyacides, les glucuronides, des composés possédant plusieurs fonctions alcools (ou phénols) et surtout des fonctions alcools (ou phénols) vicinales. On peut citer dans cette catégorie les glycols, les catéchols et les catécholamines (DOPA, adrénaline), les polyéthylèneglycols, les polyols tel que le glycérol, les monosaccharides (L-thréose, L-ribose, sorbitol...) ; des acides phénoliques tels que l'acide gallique, l'acide 3,4-dihydroxybenzoique ou l'acide caffëique, et dérivés estérifiés ; les diacides tels que l'acide malonique ; certains composés possédant une géométrie particulière propre à stabiliser en milieu aqueux les complexes de silyls, telles que les tropolones (ex: la thujaplicine).

En conséquence, une caractéristique importante de l'invention est qu'au moins un des radicaux liés à l'atome de silicium soit à même de libérer par hydrolyse, un composé stabilisant tel que défini ci-dessus.

In vivo, la réaction d'hydrolyse peut avoir lieu spontanément en l'absence de catalyseur. La vitesse de formation du silyl va dépendre alors de plusieurs facteurs:

- le pH : un environnement acide ou basique augmente fortement la vitesse de réaction
- la présence d'un catalyseur enzymatique qui va augmenter spécifiquement la vitesse d'hydrolyse de certaines liaisons hydrolysables
- la structure chimique du précurseur
- la quantité d'eau présente, la température
- la présence de sels ou de molécules biologiques possédant un hydrogène actif.

Une des caractéristiques des précurseurs, c'est qu'ils sont solubles dans les milieux apolaires : solvants organiques, huiles, silicones... contrairement à la forme "silyl" très polaire, soluble en phase aqueuse et insoluble dans les huiles. Dans la mesure où la plupart des précurseurs sont rapidement hydrolysés au contact de l'eau pour former des silyls, ces produits offrent une grande souplesse pour la formulation, notamment lors de l'incorporation dans une émulsion.

On pourra donc utiliser les précurseurs dans les formulations anhydres. Le silyl sera seulement formé in situ, au contact de l'organe cible. Ceci présente des avantages dans la mesure où les produits lipophiles traversent mieux certaines barrières biologiques.

La modification de polarité peut également être utilisée pour que le silyl actif conserve après hydrolyse une affinité pour les milieux apolaires. Ceci est le cas avec le 2,2-éthoxy, n-octyl-4-oxobenzo-1,3-dioxa-2-silane.

Les formes des précurseurs lipophiles sont, en l'absence d'eau, stables sur un large éventail de température et à toute concentration. Ils peuvent être utilisés purs alors que les formes de silyl correspondantes ne sont stables qu'à forte dilution dans un intervalle de température plus étroit et demandent l'addition d'un stabilisant ou complexant.

Une des caractéristiques importantes de ces produits est qu'il est possible de moduler la sensibilité du précurseur à l'hydrolyse, en jouant sur sa structure chimique. En effet, si certains précurseurs sont hydrolysés par le seul contact avec l'humidité atmosphérique, il est parfaitement possible, à l'inverse, d'obtenir des précurseurs stables en milieu aqueux dans des conditions de pH proches de la neutralité. Ces dernières peuvent être utiles lorsqu'une libération sélective du principe actif est recherchée, par exemple lors d'une hydrolyse au niveau stomacal.

Si l'hydrolyse du précurseur n'est pas trop rapide, il est possible d'obtenir une prolongation de l'effet. Cet "effet retard" s'applique tout particulièrement aux précurseurs déprotégés séquentiellement (les liaisons hydrolysables sont de nature chimique différente).

Accessoirement, la formation du précurseur peut contribuer à stabiliser certains des composés utilisés comme groupements protecteurs P du silyl, ce qui peut être utile lorsque P-H possède une activité biologique.

Par conséquent, les précurseurs pourront être utilisés en émulsion dans des lipides, sous forme de gel gras, de crème, de pommade pour des administrations topiques, aussi bien pour des applications thérapeutiques que cosmétiques, ou sous forme de gélules pour l'administration par voie orale dans des traitements thérapeutiques, ou encore sous forme d'injections intramusculaires.

De façon générale, les composés selon l'invention pourront être utilisés dans de nombreuses applications mettant en jeu les propriétés des formes de "silyl", à savoir les propriétés thérapeutiques, diététiques ou cosmétiques découlant de leur activité anti-inflammatoire, régénératrice, anti-dégénérescence, normalisatrice, stimulatrice métabolique, anti-radicalaire et anti-glycation, et de façon générale l'activité de stimulation des défenses de l'organisme.

Les exemples qui suivent sont illustratifs (mais non limitatifs) des formulations utilisées comme médicaments ou produits cosmétiques pour mettre en évidence les activités énoncées ci-dessus.

EXEMPLE 1 :

*Activité régénératrice*

*Restructuration des microvaisseaux*

Dans cet exemple, on a formulé des gélules pour administration orale avec du diméthyldidécanoyloxysilane (M.M.=400,67), correspondant à un équivalent SiOH de 11,5%, afin d'améliorer les microcirculations veineuse et lymphatique en agissant sur les tuniques capillaires.
On a fabriqué des gélules n°5 avec une dissolution de l'actif à 40% dans de l'huile d'Arachide, ce qui correspond à un apport de 0,03 g par gélule de silyl. Ces gélules ont été utilisées en diététique par des personnes se plaignant de jambes lourdes. L'administration a été d'une à deux gélules par jour. Les tests ont été faits sur quinze personnes de plus de 70 ans.
Douze personnes ont fait part de la disparition des douleurs et d'une réduction de l'oedème vespéral des chevilles. Sur ces douze personnes, trois ont retrouvé (disent-elles) des jambes de 20 ans.
Par contre les trois autres personnes intégrées dans cet essai n'ont eu aucun résultat, ce qui correspondrait à 20% d'échec.

EXEMPLE 2 :

*Activités anti-inflammatoire, anti-oedémateuse, analgésique et réparatrice*

*Composition dermo-pharmaceutique*

L'expérimentation a été faite sur des érythèmes graves en utilisant du 2-oxo-benzyloxy-diméthylsilane de M. M.=180,28, ce qui équivaut à 25% de SiOH.
2-oxo-benzyloxy-diméthylsilane a été dilué à 25% dans de l'Isostéraryl benzoate qui ultérieurement a été incorporé à raison de 10% dans un gel gras, et ce, afin de rétablir le film hydrolipidique protecteur et de stimuler la régénération de la peau par le silicium. Cet apport silicique étant très important a permis d'intervenir positivement sur des érythèmes plus ou moins oedématiés et sur la douleur consécutive aux lésions des tissus cutanés.
Le gel testé a pour formule :

| | |
|---|---|
| 2-oxo-benzyloxy-diméthylsilane à 25% | 10,00 |
| Lanolate de sodium | 12,00 |
| Stéarate d'aluminium | 1,00 |
| Huile de vaseline | 100,00 |

L'essai a été fait sur 10 personnes atteintes d'érythème solaire grave avec la liberté d'appliquer le gel aussi souvent que la personne le désirait.
On a obtenu dans 80% des cas, une cessation de la douleur et une réduction de l'oedème dans moins de 6 heures, et une réduction de la rougeur de plus de 50% en 24 heures, ce qui est très positif.
D'autre part, ce gel gras a été utilisé après des séances de cobaltothérapie, sur des muqueuses.
on a observé une protection superficielle de ces muqueuses avec une diminution de la douleur et de l'inflammation.

EXEMPLE 3 :

*Action anti-inflammatoire*

*Traitement des tendinites du sportif*

Il a été procédé à la réalisation d'une crème de massage grasse. Les massages ont été effectués par des masseurs-kinésithérapeutes, les essais ont été faits sur des Tennis-Elbow.
Le composé testé a été le 2-oxo-benzyl-oxy diméthylsilane de M.M.=180,28, ce qui équivaut à 25% de SiOH.
On a simplement intégré du produit pur à 8% dans une lano-vaseline qui est une crème excessivement grasse permettant un massage long jusqu'à quasi-pénétration.
Les résultats de l'expérimentation portant sur douze personnes atteintes de Tennis-Elbow sont les suivants :

Le malade doit respecter un repos de 14 à 21 jours, durant lequel 3 fois par jour, il doit masser la partie douloureuse au moyen de la crème.

Sur les 12 malades, 4 ont vu disparaître leur douleur et ont pu reprendre leur activité au bout de 6 jours, 5 au bout de 12 jours, 1 au bout de 14 jours, et 2 au bout de 16 jours.

Au moyen de cette crème, il a été possible d'intervenir sur des tendinopathies de l'épaule, soit sous scapulaire, soit sus-épineux, soit long biceps.

Ces tendinopathies se manifestaient par une épaule douloureuse simple (EDS) ou une épaule aiguë, hyperallergique (EAH).

L'expérimentation a porté sur 12 malades qui ont été traités 3 fois par jour.

2 malades présentant une tendinopathie (EDS) ont été soulagés au bout de 5 jours, 3 au bout de 9 jours, 1 malade atteint d'une tendinopathie (EHA) a été soulagé au bout de 7 jours, 1 au bout de 12 jours, 3 au bout de 15 jours et 2 n'ont pas vu leur état modifié.

EXEMPLE 4 :

*Action normalisatrice*

*Traitement de l'asthénie*

Dans cet exemple on a utilisé l'Ascorbosilyl ou diméthylsilyl -2, 3, 5, 6 ascorbate, ayant pour formule $C_{10}H_{16}O_6Si_2$. On a M.M.=288,4, donnant 24,56% de Si, ce qui équivaut à 32% de SiOH.

Ce composé a été étudié dans la remise en forme, la lutte contre les attaques du vieillissement et les coups de fatigues.

La formulation a été réalisée en diététique par administration *per os* de gélules dosées à 0,5 g d'actif.

L'intérêt est d'apporter des quantités très importantes de silyl ; soit jusqu'à 0,19 g de SiOH et 0,31 g d'acide ascorbique par gélule.

On a expérimenté une forme diluée à 20% d'ascorbosilyl dans l'huile de soja, ce qui donne 0,04 g en silyl et 0,07 g en acide ascorbique par unité de prise. L'administration est de 1 à 3 gélules par jour.

Ce composé permet d'apporter *per os* des quantités qui seraient impossibles dans le cas d'un silyl classique obtenu par complexation.

La prise de ces gélules a donné des résultats très rapides dans certains cas avec un retour de la forme physique, dès le 8è jour. Dans d'autres cas, la réponse n'est intervenue que 3 ou 4 mois plus tard, mais avec dans tous les cas des modifications du comportement, un retour de la sensation de bien être et une meilleure adéquation à la vie quotidienne.

EXEMPLE 5 :

*Activité anti-radicalaire*

*Utilisation comme cosmétiques colorés*

Le composé testé a été l'ethoxyméthyl silyl-2-oxo octanoate de M.M. = 246,7, ce qui équivaut à 19% de SiOH.

Ce composé a été dilué dans de l'huile d'Onagre, à raison de 45%.

Cette solution a été utilisée à raison de 7% dans des mascaras et des rouges à lèvres, ce qui correspond à un apport de 1% en SiOH.

Ces cosmétiques avaient pour but d'assurer régénération, protection des muqueuses et des épidermes sensibles.

Ceci est prouvé par le test suivant qui mesure le pourcentage d'inhibition du taux d'oxydation témoin.

Le test consiste à produire des radicaux libres oxygénés par voie enzymatique (action de la xanthine oxydase sur l'acétaldéhyde) et à comparer la résistance des cellules (cultivées en présence ou en l'absence d'éthoxyméthylsilyl-2-oxo-octanoate) au contact de ces radicaux. L'addition de radicaux libres, au sein d'une culture cellulaire induit une cytotoxicité. Cette toxicité se traduit par une lyse cellulaire avec une augmentation de la lactate déshydrogénase (LDH) cellulaire. L'évaluation de la résistance cellulaire au stress radicalaire est obtenue par le dosage de l'activité de la LDH en spectrophotométrie UV. On mesure, si DO désigne la densité optique, l'activité de la LDH au moyen du rapport suivant

$$\frac{\text{DO témoin - DO essai}}{\text{DO essai}} \times 100$$

*Résultats :*

On observe un effet protecteur très sensible de silyl. Le système peroxydatif utilisé a produit des ions superoxydes et du peroxyde d'hydrogène. L'étude des résultats montre que le silyl induit une protection contre la lyse cellulaire spontanée (activité de la LDH diminuée de 73%). Cette protection persiste et s'intensifie (diminution de la LDH de 77%) en présence d'un stress radicalaire provoqué.

EXEMPLE 6:

*Activité anti-glycation, anti-radicalaire*

*Traitement de la cataracte*

En ophtalmologie comme médicament anti-catharacte on a utilisé du diméthylsilyl-2, 3, 5, 6 ascorbate (ou ascorbosilyl) de formule $C_{10}H_{16}O_6Si_2$ avec M.M.=288,4, soit 24,56% de Si, ce qui équivaut à 32% de SiOH.
L'ascorbosilyl est incorporé à raison de 6,5% dans une pommade ophtalmique composée d'un mélange de vaseline et d'huile de vaseline.
L'ascorbosilyl après hydrolyse au contact du liquide lacrymal et de la cornée agit progressivement en tant qu'anti-radicalaire, empêchant la peroxydation des lipides membranaires cellulaires.
Il contribue également à restructurer les protéoglycanes et les muccopolysaccharides constitutifs de la cornée.
Il est à noter que la présence de l'acide ascorbique potentialise l'action antiradicalaire puisque c'est un constituant naturel de l'oeil.
L'activité anti-radicalaire propre à un médicament anti-catharacte a été vérifiée par le test suivant :
Le test consiste à produire des radicaux libres oxygénés par voie enzymatique (action de la xanthine oxydase sur l'acétaldéhyde) et à comparer la résistance des cellules (cultivées en présence ou en l'absence de diméthylsilyl-2, 3, 5, 6 ascorbate) au contact de ces radicaux.
L'addition de radicaux libres, au sein d'une culture cellulaire induit une cytoxicité. Cette toxicité se traduit par une lyse cellulaire avec une augmentation de la lactate déshydrogénase (LDH) cellulaire. L'évaluation de la résistance cellulaire au stress radicalaire est obtenue par le dosage de l'activité de la LDH en spectrophotométrie UV.

*Résultats* :

On observe un effet protecteur très sensible du diméthylsilyl-2, 3, 5, 6 ascorbate. L'activité du silyl est estimée à partir des variations d'activité de la LDH par rapport au témoin d'analyse. Le système peroxydatif utilisé produit des ions superoxydes et du peroxyde d'hydrogène. L'étude des résultats montre que le diméthylsilyl-2, 3, 5, 6 ascorbate induit une protection contre la lyse cellulaire spontanée (activité de la LDH diminuée de 67%). Cette protection persiste et s'intensifie (diminution de la LDH de 79%) en présence d'un stress radicalaire provoqué.

## Revendications

1. Composé à base silicium **caractérisé en ce qu'**il libère par hydrolyse in vivo un composé silylé biologiquement actif et au moins un stabilisant, ledit composé ayant la formule générale suivante:

a) 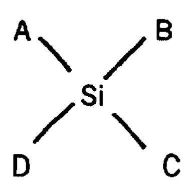

b) 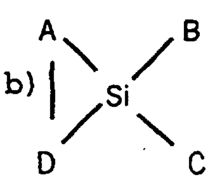

dans laquelle:

- A et D sont des radicaux tels que leur produits d'hydrolyse A-H et/ou D-H est un stabilisant choisi dans le groupe consistant en :
  acides carboxyliques simples possédant une chaîne carbonée de plus de cinq atomes de carbone, phénol, hydroxyacides carboxyliques tels que les alpha et les bêta hydroxyacides, les glucuronides, composés comportant plusieurs fonctions alcools ou phénols et surtout des fonctions alcools ou phénols vicinales tels que les glycols, les catéchols, et les catécholamines, les polyéthylène-glycols, les polyols tels que le glycérol, les monosaccharides tels que le L-thréose, L-ribose ou sorbitol, les acides phénoliques tels que l'acide caféique et leurs dérivés estérifiés, les diacides tels que l'acide malonique, et certains composés possédant une géométrie particulière tels que le tropolone,

- B est un radical tel que son produit d'hydrolyse est choisi parmi les composés que représentent A-H et D-H ou un radical alkyl,

- C est un radical hydrocarboné tel qu'un groupement alkyle alkényle, alkyne, arylalkyle ou aryle, à l'exception d'un phényle.

**2.** Composé à base de silicium **caractérisé en ce qu'**il libère par hydrolyse in vivo un composé silylé biologiquement actif et au moins un stabilisant, ledit composé étant le caproyldiéthoxysilane.

**3.** Composé à base de silicium **caractérisé en ce qu'**il libère par hydrolyse in vivo un composé silylé biologiquement actif et au moins un stabilisant, ledit composé étant choisi parmi les composés suivants: 2-oxo-benzyloxy-éthoxyméthylsilane, éthoxyméthysilyl-2-oxo-octanoate, 2,2-éthoxyméthyl-4-oxo-5-méthyl-1,3-dioxa-2-cyclosilane, 2,2 méthyl,tertbutoxy-4-oxo-1,3-dioxa-2-silane et 2,2-éthoxy,n-octyl-4-oxobenzo-1,3-dioxa-2-silane.

**4.** Application du composé selon la revendication 1 à 3, pour obtenir une composition thérapeutique, diététique ou cosmétique ayant une activité anti-inflammatoire, régénératrice, anti-dégénérescente, normalisatrice, stimulatrice du métabolisme, anti-radicalaire ou anti-glycation, ou de façon générale une activité de stimulation des défenses de l'organisme humain ou animal.

**5.** Composition thérapeutique résultant de l'application selon la revendication 4, ayant une activité anti-inflammatoire pour le traitement de l'arthrose, à base de diméthylsilyl-L-hydroxyproline.

**6.** Composition thérapeutique selon la revendication 5 dans laquelle le diméthylsilyl-L-hydroxyproline est administré sous forme de gélules.

**7.** Composition thérapeutique résultant de l'application selon la revendication 4, ayant une activité régénératrice pour la restructuration des microvaisseaux, à base de diméthyldidécanoyloxysilane.

**8.** Composition thérapeutique selon la revendication 7 dans laquelle le diméthyldidécanoyloxysilane est administré sous forme de gélules.

**9.** Composition thérapeutique résultant de l'application selon la revendication 4, ayant une activité anti-inflammatoire, anti-oedémateuse, analgésique et réparatrice pour le traitement des érythèmes, à base de 2-oxo-benzyloxy-diméthylsilane.

**10.** Composition thérapeutique selon la revendication 9 dans laquelle le 2-oxo-benzyloxy-diméthylsilane est utilisé sous forme de gel gras.

**11.** Composition thérapeutique résultant de l'application selon la revendication 4, ayant une activité anti-inflammatoire pour le traitement des tendinites du sportif, à base de 2-oxo-ébenzyloxy-diméthylsilane.

**12.** Composition thérapeutique selon la revendication 11 dans laquelle le 2-oxo-benzyloxy-diméthylsilane est utilisé dans une crème de massage grasse.

**13.** Composition thérapeutique résultant de l'application selon la revendication 4, ayant une activité anti-radicalaire et anti-glycation pour le traitement de la cataracte, à base de diméthylsilyl-2,3,5,6 ascorbate.

**14.** Composition thérapeutique selon la revendication 13, dans laquelle le diméthylsilyl-2,3,5,6 ascorbate est incorporé dans une pommade ophtalmique.

**15.** Composition diététique résultant de l'application selon la revendication 4, ayant une activité normalisatrice pour

la remise en forme, les attaques contre le vieillissement et les coups de fatigue, à base de diméthylsilyl-2,3,5,6 ascorbate.

**16.** Composition diététique selon la revendication 15 dans laquelle le diméthylsilyl-2,3,5,6 ascorbate est administré sous forme de gélules.

**17.** Composition cosmétique résultant de l'application selon la revendication 4, ayant une activité anti-radicalaire, ladite composition utilisée comme mascara ou rouge à lèvres étant à base d'éthoxyméthyl silyl-2-oxo-octanoate.

**Patentansprüche**

**1.** Verbindung auf der Basis von Silicium, **dadurch gekennzeichnet, dass** sie durch in-vivo-Hydrolyse eine biologisch aktive silylierte Verbindung und mindestens einen Stabilisator freisetzt, wobei diese Verbindung die folgende allgemeine Formel besitzt:

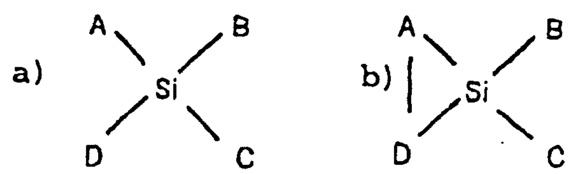
a) b)

in welcher:

- A und D solche Reste sind, dass ihre Hydrolyseprodukte A-H und/oder D-H ein Stabilisator sind, der aus der aus den folgenden Substanzen bestehenden Gruppe ausgewählt ist:
  einfache Carboxylsäuren mit einer Kohlenstoffkette von mehr als fünf Kohlenstoffatomen, Phenol, Hydroxycarbonsäuren, wie z.B. alpha- und beta-Hydroxysäuren, Glucuronide, Verbindungen mit mehreren Alkohol- oder Phenolfunktionen und vor allem vicinalen Alkohol- oder Phenolfunktionen, wie z.B. Glykole, Catechole, und Catecholamine, Polyethylenglykole, Polyole, wie z.B. Glycerin, Monosaccharide, wie z.B. L-Threose, L-Ribose oder Sorbitol, Phenolsäuren, wie z.B. Kaffeesäure und ihre veresterten Derivate, zweibasische Säuren, wie z.B. Malonsäure, und gewisse Verbindungen mit einer besonderen Geometrie, wie z.B. Tropolon,

- B ein solcher Rest ist, dass sein Hydrolyseprodukt aus den durch A-H und D-H dargestellten Verbindungen oder einem Alkylrest ausgewählt ist,

- C ein Kohlenwasserstoffrest ist, wie z.B. eine Alkylalkenyl-, Alkin-, Arylalkyl- oder Arylgruppe mit Ausnahme einer Phenylgruppe.

**2.** Verbindung auf der Basis von Silicium, **dadurch gekennzeichnet, dass** sie durch in-vivo-Hydrolyse eine biologisch aktive silylierte Verbindung und mindestens einen Stabilisator freisetzt, wobei diese Verbindung Caproyldiethoxysilan ist.

**3.** Verbindung auf der Basis von Silicium, **dadurch gekennzeichnet, dass** sie durch in-vivo-Hydrolyse eine biologisch aktive silylierte Verbindung und mindestens einen Stabilisator freisetzt, wobei diese Verbindung aus den folgenden Verbindungen ausgewählt ist: 2-Oxobenzyloxyethoxymethylsilan, Ethoxymethylsilyl-2-oxooctanoat, 2,2-Ethoxymethyl-4-oxo-5-methyl-1,3-dioxa-2-cyclosilan, 2,2-Methyl-tert.butoxy-4-oxo-1,3-dioxa-2-silan und 2,2-Ethoxy-n-octyl-4-oxobenzo-1,3-dioxa-2-silan.

**4.** Verwendung der Verbindung nach Anspruch 1 bis 3 zur Herstellung einer therapeutischen, diätetischen oder kosmetischen Zusammensetzung mit entzündungshemmender, regenerierender, antidegenerierender, normalisierender, stoffwechselanregender Wirkung, mit einer Wirkung gegen freie Radikale oder Glykation oder allgemein mit einer die Abwehrkräfte des menschlichen oder tierischen Organismus anregenden Wirkung.

**5.** Durch die Verwendung nach Anspruch 4 erhaltene therapeutische Zusammensetzung mit einer entzündungshemmenden Wirkung für die Behandlung der Arthrose auf der Basis von Dimethylsilyl-L-hydroxyprolin.

**6.** Therapeutische Zusammensetzung nach Anspruch 5, bei der das Dimethylsilyl-L-hydroxyprolin in Form von Gelatinekapseln verabreicht wird.

**7.** Durch die Verwendung nach Anspruch 4 erhaltene therapeutische Zusammensetzung mit einer regenerierenden Wirkung für die Restrukturierung der Mikrogefäße auf der Basis von Dimethyldidecanoyloxysilan.

**8.** Therapeutische Zusammensetzung nach Anspruch 7, bei der das Dimethyldidecanoyloxysilan in Form von Gelatinekapseln verabreicht wird.

**9.** Durch die Verwendung nach Anspruch 4 erhaltene therapeutische Zusammensetzung mit einer entzündungshemmenden, antiödematösen, analgetischen und rückbildenden Wirkung für die Behandlung von Erythemen auf der Basis von 2-Oxobenzyloxy-dimethylsilan.

**10.** Therapeutische Zusammensetzung nach Anspruch 9, bei der das 2-Oxobenzyloxydimethylsilan in Form von Fettgel verwendet wird.

**11.** Durch die Verwendung nach Anspruch 4 erhaltene therapeutische Zusammensetzung mit einer entzündungshemmenden Wirkung für die Behandlung von Tendinitis bei Sportlern auf der Basis von 2-Oxobenzyloxydimethylsilan.

**12.** Therapeutische Zusammensetzung nach Anspruch 11, bei der das 2-Oxobenzyloxydimethylsilan in einer Massage-Fettcreme verwendet wird.

**13.** Durch die Verwendung nach Anspruch 4 erhaltene therapeutische Zusammensetzung mit einer Wirkung gegen freie Radikale und Glykation für die Behandlung des grauen Stars auf der Basis von Dimethylsilyl-2,3,5,6-ascorbat.

**14.** Therapeutische Zusammensetzung nach Anspruch 13, bei der das Dimethylsilyl-2,3,5,6-ascorbat in eine Augensalbe eingearbeitet ist.

**15.** Durch die Verwendung nach Anspruch 4 erhaltene diätetische Zusammensetzung mit einer normalisierenden Wirkung für die Wiederherstellung der Leistungsfähigkeit, gegen Altern und Erschöpfung auf der Basis von Dimethylsilyl-2,3,5,6-ascorbat.

**16.** Diätetische Zusammensetzung nach Anspruch 15, bei der das Dimethylsilyl-2,3,5,6-ascorbat in Form von Gelatinekapseln verabreicht wird.

**17.** Durch die Verwendung nach Anspruch 4 erhaltene kosmetische Zusammensetzung mit einer Wirkung gegen freie Radikale, wobei diese eine als Wimperntusche oder Lippenstift verwendete Zusammensetzung auf der Basis von Ethoxymethylsilyl-2-oxooctanoat ist.

## Claims

**1.** Silicon compound **characterized in that** it releases by hydrolysis in vivo a biologically active silyl compound and at least one stabilizer, said compound having the general formula :

a) 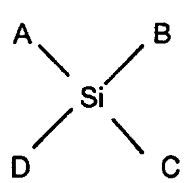

b) 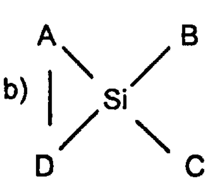

in which :

- A and D are radicals so that their hydrolysis product A-H and/or D-H is a stabilizer selected from the group consisting of :

  simple carboxylic acids having a carbon chain of more than five carbon atoms, phenols, hydroxy carboxylic acid such as the alpha and beta hydroxy acids, the glucuronides, compounds possessing several alcohol or phenol functions and above all vicinal alcohol (or phenol) functions such as the glycols, the catechols and the catecholamine, the polyethylene-glycols, the polyols such as glycerol, the monosaccharides such as L-threose, L-ribose, or sorbitol, the phenolic acids such as caffeic acid and esterified derivatives, the diacids such as malonic acid, and some compounds possessing a particular geometry such as the tropolone,
- B is a radical so that his hydrolysis product is selected among the compounds which are represented by A-H and D-H or an alkyl,
- C is an hydrocarbon radical such as an alkyl, alkenyl, alkyn, arylalkyl or aryl group, excepted a phenyl.

**2.** Silicon compound **characterized in that** it releases by hydrolysis in vivo a biologically active silyl compound and at least one stabilizer, said compound being the caproyldiethoxysilane.

**3.** Silicon compound **characterized in that** it releases by hydrolysis in vivo a biologically active silyl compound and at least one stabilizer, said compound being selected among the following compounds : 2-oxo-benzyloxyethoxymethylsilane, ethoxymethylsilyl-2-oxo-octanate, 2,2-ethoxymethyl-4-oxo-5-methyl-1,3-dioxa-2-cyclosilane, 2,2-methyl,tertbutoxy-4-oxo-1,3-dioxa-2-silane and 2,2-ethoxy,n-octyl-4-oxobenzo-1,3-dioxa-2-silane.

**4.** Application of the composition according to one of claims 1 to 3 in order to obtain a therapeutic, dietetic or cosmetic composition having an anti-inflammatory, regenerating, anti-degeneration, normalizer, metabolic stimulator, anti-free radical and anti-glycation, or generally speaking an activity of stimulation of the human or animal organism defences.

**5.** Therapeutic composition resulting from the application according to claim 4, having an anti-inflammatory activity for the dimethylsilyl-L-hydroxyproline treatment of arthrosis.

**6.** Therapeutic composition according to claim 5 in which the dimethylsilyl-L-hydroxyproline is administered under the form of capsules.

**7.** Therapeutic composition resulting from the application according to claim 4, having a regenerating activity with the dimethyldidecanoyloxysilane for the microvessels restructuration.

**8.** Therapeutic composition according to claim 7 in which the dimethyldidecanoyloxysilane is administered under the form of capsules.

**9.** Therapeutic composition resulting from the application according to claim 4 having an anti-inflammatory, anti-oedematic, analgesic and repairing activity for the erythema treatment with 2-oxo-benzyloxy-dimethylsilane.

**10.** Therapeutic composition according to claim 9 in which the 2-oxo-benzyloxy-dimethylsilane is used under the form of an oily gel.

**11.** Therapeutic composition resulting from the application according to claim 4, having an anti-inflammatory activity for the sport's man tendinitis treatment with the 2-oxo-benzyloxy-dimethylsilane.

**12.** Therapeutic composition according to claim 11 in which 2-oxo-benzyloxy-dimethylsilane is used in an oily cream for massage.

**13.** Therapeutic composition resulting of the application according to claim 4, having an anti-free radical and anti-glycation activity for the cataract treatment with the dimethylsilyl-2,3,5,6 ascorbate.

**14.** Therapeutic composition according to claim 13, in which the dimethylsilyl-2,3,5,6 ascorbate is incorporated in an ophthalmic ointment.

**15.** Dietetic composition resulting from the application according to claim 4, having the normalizating activity for the reshaping, the struggle against the ageing process and the tired spells, with the dimethylsilyl-2,3,5,6 ascorbate.

**16.** Dietetic composition according to claim 15 in which the dimethylsilyl-2,3,6 ascorbate is administered under the form of capsules.

**17.** Cosmetic composition resulting from the application according to claim 4, having an anti-free radical activity, this composition is used as mascara or lipstick with the ethoxymethyl silyl-2-oxo-octanoate.